# EUROPEAN PATENT APPLICATION

(11) **EP 4 599 873 A1**
(43) Date of publication of application: **13.08.2025**
(21) Application number: 24156167.9
(22) Date of filing: 06.02.2024
(51) Int. Cl.: A61M 15/00, A61M 11/00

(54) **ACCESSORIES FOR INHALERS**

(71) Applicant: PLASTIAPE S.p.A., 23875 Osnago (Lecco) (IT); Amiko S.R.L., 20146 Milano (IT)
(72) Inventor: CITTERIO, Mauro, 23875 Osnago (IT); GRINOVERO, Martijn, 20146 Milano (IT); PONTI, Luca, 20146 Milano (IT); FATO, Alessandro, 20146 Milano (IT)
(74) Representative: Bryers Intellectual Property Ltd

(57) **Abstract**

An accessory (100) for obtaining inhalation data of an inhaler (10) is provided. The accessory is integrally formed with or attached to the inhaler. The accessory comprises: one or more airflow-induced vibration generators (110a, 110b) configured to generate mechanical oscillations. The accessory further comprises a monitoring system comprising one or more accelerometer sensors (135) configured to receive, from the airflow-induced vibration generator, the generated mechanical oscillations and configured to generate acceleration signals based on the received mechanical oscillations. The accessory further comprises an electronic processing unit (350) electrically connected to the accelerometer sensors, configured to receive, from the accelerometer sensor, the generated acceleration signals and configured to extract inhalation data from the received acceleration signal.

## Description

The present disclosure relates to accessories for inhalers.

### BACKGROUND

The inhalers are the main treatment means for patients with pulmonary diseases such as asthma and Chronic Obstructive Pulmonary Disease (COPD). The three main types of inhalers are: the "metered-dose inhalers" or MDIs, the "dry powder inhalers" or DPIs and the "soft mist inhalers" or SMIs.

In all these types of inhalers, the result of a respiratory medication therapy is, among others, depending on the correct inhalation technique. A proper use of the inhaler is essential, particularly in situations where a patient is required to regularly self-medicate to manage his disease condition.

Current clinical evidence suggests that inhaled therapy often fails to achieve expected results due to poor inhalation technique by the patient.

There have been attempts at addressing these problems with inhalers combined with accelerometer-based devices which can be effective in generating inhalation data directly from inhalers by sensing mechanical oscillations during inhalation of the subject. However, such devices may not be the best option for inhalers that do not generate clear and distinguishable mechanical oscillations.

For example, certain inhalers may exhibit weaker mechanical oscillations due to reduced airflow turbulence during inhalation, and other inhalers may have designs that limit the propagation of mechanical oscillations through the inhaler materials. The issue of weak mechanical oscillations is further amplified in cases of low airflow during inhalation of the subject, making it challenging to accurately measure airflow properties using accelerometer-based devices.

Examples of the present disclosure seek to at least partially reduce one or more of the aforementioned problems.

### SUMMARY

According to a first aspect, an accessory for obtaining inhalation data from an inhaler is provided. The accessory is integrally formed with or attached to the inhaler. The accessory comprises: one or more airflow-induced vibration generators configured to generate mechanical oscillations. The accessory further comprises a monitoring system comprising one or more accelerometer sensors configured to receive, from the airflow-induced vibration generator, the generated mechanical oscillations and configured to generate acceleration signals based on the received mechanical oscillations. The monitoring system further comprises an electronic processing unit electrically connected to the accelerometer sensors, configured to receive, from the accelerometer sensor, the generated acceleration signals and configured to extract inhalation data from the received acceleration signals.

According to this aspect, an accessory for obtaining and / or generating inhalation data related to an inhaler is provided. The accessory is an accelerometer-device with simple and inexpensive airflow-induced vibration generators that generate mechanical oscillations (and thus vibration signals) with specific characteristics that enable the accurate and reliable measurement of inhalation data including airflow properties, even in cases of low airflow during inhalation of the subject using the inhaler. The airflow-induced vibration generators respond to the airflow passing through the inhaler to produce detectable and measurable mechanical oscillations, providing highly accurate measurement of inhalation data and airflow properties without impacting cost-effectiveness.

In a further aspect, a kit is provided. The kit includes an accessory according to the first aspect and an inhaler.

In some examples, the inhaler is one of the following: a metered-dose inhaler, a dry powder inhaler, or a soft mist inhaler.

The inhaler may thus be used with the three main types of inhalers, namely the "metered-dose inhalers" or MDIs, the "dry powder inhalers" or DPIs and the "soft mist inhalers" or SMIs.

### BRIEF DESCRIPTION OF DRAWINGS

Non-limiting examples of the present disclosure will be described in the following, with reference to the appended drawings, in which:
Figure 1 schematically illustrates a perspective view of an example of an inhaler and an accessory installed on the inhaler;
Figure 2 schematically illustrates an exploded view of the inhaler and the accessory installed on the inhaler shown in figure 1;
Figure 3 schematically illustrates a top cross-sectional view of the accessory shown in figure 1 and figure 2;
Figures 4a - 4d illustrate various steps of an example of method for using the inhaler together with the inhaler shown in the previous figures;
Figure 5 schematically illustrates a perspective view of another example of an inhaler and an accessory installed on the inhaler, wherein the inhaler in a closed configuration;
Figure 6 schematically illustrates a perspective view of the inhaler and the accessory shown in figure 5, wherein the inhaler is in an open configuration;
Figure 7 schematically illustrates an exploded view of the accessory shown in figures 5 and 6;
Figure 8 schematically illustrates an example of an attachment detection unit and an opening detection unit in the inhaler and the accessory shown in figures 5 - 7;
Figure 9 schematically illustrates a perspective view of yet another inhaler;
Figure 10 schematically illustrates a perspective view of the inhaler shown in figure 9 including an example of an accessory.

### DETAILED DESCRIPTION OF EXAMPLES

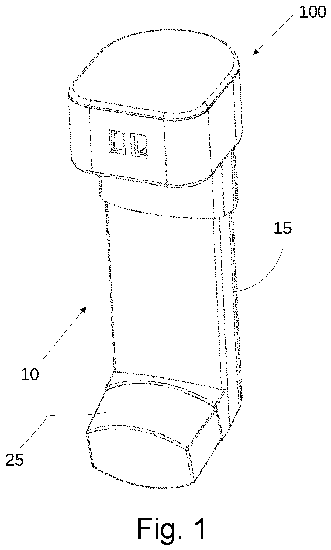

Figure 1 schematically illustrates a perspective view of an example of an inhaler 10 and an example of an accessory 100 installed on the inhaler. In this particular example, the inhaler 10 is a metered-dose inhaler. However, in some other examples, the inhaler 10 may be a dry powder inhaler or a soft mist inhaler.

The inhaler 10 comprises a body 15 for receiving a canister (not visible in this figure). The canister may be produced in aluminum or stainless steel. The body 15 comprises a mouthpiece 25 through which aerosolized canister content can be dispensed. The formulation itself may be made up of a drug, a liquefied gas propellant and, in many cases, stabilizing excipients.

The inhaler 10 further comprises a metering valve (not visible) which allows a metered quantity of the formulation to be dispensed with each actuation. The inhaler may further comprise a cap (not shown) for covering the mouthpiece 25. In this example, the accessory 100 may act as an actuator which allows the user to operate the device and directs the aerosol into the user's lungs. The body 15 may be equipped either with a digital or with a mechanical dose counter or dose indicator.

Typically, a metered-dose inhaler may be operated as follows: the user uses the inhaler by pressing down on the top of the inhaler, with his thumb supporting the lower portion of the body. Actuation of the device releases a single metered dose of the formulation which contains the medication either dissolved or suspended in the propellant. Breakup of the volatile propellant into droplets, followed by rapid evaporation of these droplets, results in the generation of an aerosol comprising micrometer-sized medication particles that are then inhaled. The operation of this particular inhaler 10 will be described with more detail later on.

As can be seen in figure 1, the mouthpiece 25 may be integrally formed with the body 15. However, in some other examples, the mouthpiece 25 may be detachably connected to the body 15 such that the mouthpiece 25 can be removed. For example, the mouthpiece 25 may be rotatably or slidably connected to the body 15. The mouthpiece 25 may be replaced and selected according to the desired properties.

As commented above, the accessory 100 is installed on the inhaler 10. The accessory 100 is configured for measuring airflow properties of e.g., the air flow directed to the mouth of the user provoked by the inhalation of a user via the mouthpiece 25.

In examples, the accessory 100 may be provided integrally formed with the body 15 of the inhaler. This allows for a relatively simple and more compact design. In some other examples, the accessory 100 may be provided permanently or removably attached to the body 15 of the inhaler. In case the accessory is removably attached to the inhaler 10, the accessory 100 may be re-used with a different compatible inhaler. Overall, these configurations provide a flexible and versatile accessory 100 which can be used with a variety of inhaler designs. It is noted that the attachment and detachment of the accessory 100 to the body 15 of the inhaler can be easily performed by a user and does not require any trained personnel.

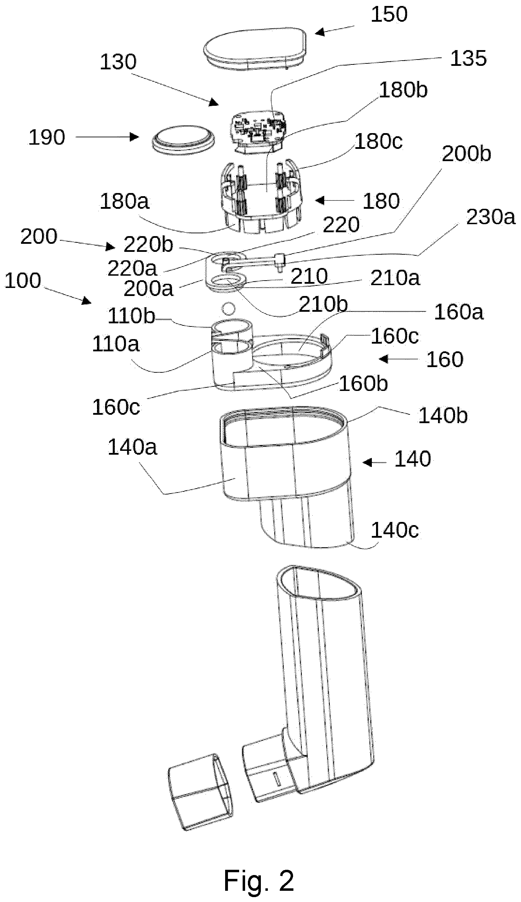

In this example, the accessory 100 is configured to be coupled to the inhaler 10. As can be seen in figure 2, the accessory 100 comprises an accessory body 140, and a separate lid 150 for closing off the top of the accessory body 140. The separate lid 150 may have e.g., a threaded coupling with the accessory body 140.

The accessory body 140 may comprise one or more sidewalls 140a extending between a top 140b and a bottom 140c of the accessory body. A space is formed in the interior of the accessory body 140 between the sidewalls.

The accessory 100 may further comprise a chassis 160, in use, disposed in the interior of 160 the accessory body 140. The chassis 160 may comprise a central opening 160a in which a central support 180 (for supporting a PCB) may be placed. The chassis 160, in this example, may comprise an annular top platform 160b and one or more outer sidewalls 160c. The chassis 160 may further comprise one or more slots 160c. The slots form female coupling elements and are adapted to receive and mate with further connectors or male coupling elements located e.g., in the central support 180. These elements may act as anti-rotation features such that, in use, the central support 180 cannot be rotated with respect to the chassis 160.

The chassis 160 may further comprise one or more airflow-induced vibration generators. In this example, two airflow-induced vibration generators 110a, 110b are provided. The airflow-induced vibration generators 110a, 110b may be attached to a portion of the outer sidewalls 160c and to a portion of the annular top platform 160b. In some other examples, the airflow-induced vibration generators 110a, 110b may be integrally formed with the chassis 160. The structure and operation of the airflow-induced vibration generators 110a, 110b will be explained later on.

Following the example, the central support 180 may comprise a plurality of male (e.g., elastic) coupling members 180a and a top platform 180b. As commented above, the central support 180 may be situated in the central opening 160a and thus the central support 180 may be kept in position within the central opening 160a of the chassis 160. For example, the central support 180 may be attached to the inner walls of the central opening 160a. Moreover, the male coupling members 180a are adapted to be attached to the upper part of the canister. Specifically, the coupling members 180a are slightly deformed during insertion of the canister and therefore, once the canister is installed to the central support 180, the male coupling members are elastically and centripetally pressing onto the canister. With such an arrangement, the central support 180 may be releasably joined to the canister of the inhaler using e.g., a snap fit locking mechanism.

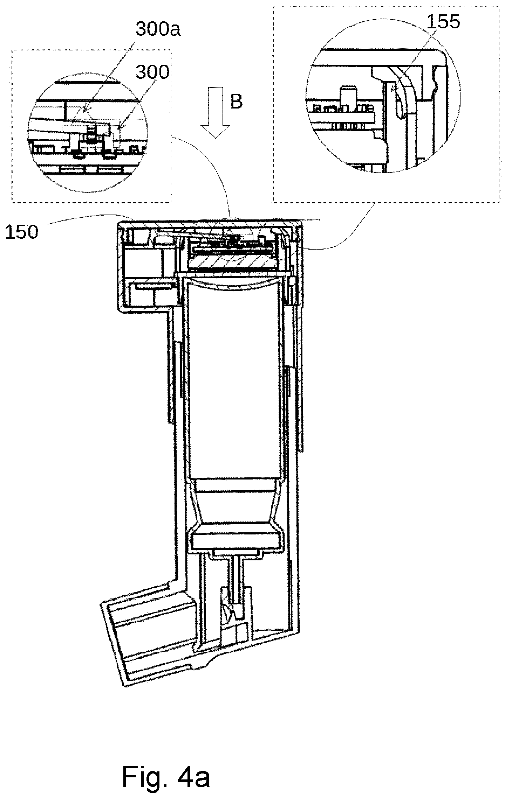

The central support 180 further comprise a plurality of elastic elements 180c. The elastic elements are configured to maintain the accessory in a first operational position, when no pressure is exerted on the top of the inhaler. However, if a user uses the inhaler by pressing down on the top of the inhaler, the elastic elements may be bent such that the accessory is displaced to a second operational position in which a single metered dose of the formulation is released. This will be described in more detail with reference to figures 4a - 4d.

Again in figure 2, the accessory 100 may further comprise a printed circuit board 130 disposed in the interior of the accessory body 140 and mechanically coupled to the top platform 180b of the central support 180 (and thus to the body 140 of the accessory). The printed circuit board 130 comprises one or more accelerometer sensors 135 electrically (and mechanically) connected to such printed circuit board 130. The printed circuit board 130 will be affected by the mechanical oscillation signals, generated by the induced vibration generators 110a, 110b, during inhalation by a user as will be explained later on. Typically, the mechanical coupling between the printed circuit board 130 and the central support 180 (and thus the accessory body 140 and the chassis 160) allows mechanical oscillations, generated by the vibration generators 110a, 110b, to propagate without (significant) attenuations e.g., from the chassis 160 of the accessory to the printed circuit board 130. Such mechanical coupling may be rigid and/or elastic, for example, it may be rigid in some places and elastic in some other places.

The accelerometers 135 may be e.g., MEMS accelerometers. Each of the accelerometers may be sensitive to accelerations in one or more directions e.g., three directions. Along with the present description and claims the term "accelerometer" is to be understood as a sensor that translates (dynamic and static) accelerations of the sensor itself into electronic signals. Specifically, the accelerometers 135 are configured to measure mechanical oscillations generated by the airflow-induced vibration generators as hereinbefore described. In this respect, the acceleration sensor may be configured to receive mechanical oscillations generated by the airflow-induced vibration generators and to generate an acceleration signal (which will be processed by a processing module).

The accelerometer may be situated at or near the airflow-induced vibration generator e.g., at a distance between 0.1 centimeters and 5 centimeters. The inventors have found that placing the accelerometer relatively close to the airflow-induced vibrator generator is an advantage. This is because vibrations tend to diminish in intensity and attenuate as they travel through different materials. The further the vibrations have to travel, the more they lose their original strength and characteristics. It is noted that there may be several factors that influence the ideal distance, like the material properties (e.g. dense materials might carry the vibrations further and with less loss compared to more porous materials) or the design of the intermediary elements.

In examples, the accelerometer sensors may comprise a sampling frequency equal or lower than 2 kHz, in order to accurately process frequencies in a band of interest between 100 Hz and 1 kHz, specifically between 200 Hz and 900 Hz. Advantageously, this relatively low sampling frequency allows to reliably generate inhalation data while reducing processing and memory requirements and optimizing system power consumption and cost.

A battery 190 is also provided. The battery 190 may be electrically connected to the printed circuit board 130.

The accessory 100 further comprises an internal cover 200 for, at least partially closing off the top of the airflow-induced vibration generators 110a, 110b. The internal cover extends longitudinally from a first end 200a to a second end 200b. At or near the first end 200a, it is provided a first lid 210 for at least partially closing off the top of the airflow-induced vibration generator 110a and a second lid 220 for at least partially closing off the top of the airflow-induced vibration generator 110b. Each of the lids includes an outer ring 210a, 220a and a central opening 210b, 220b.

The internal cover 200 further comprises one or more pins situated at or near the second end 200b of the internal cover. In this particular example, a first pin 230a and a second pin (not visible) are provided. The pins 230a may be configured to be mechanically attached e.g., welded to the printed circuit board 130. This way, in use, the mechanical oscillations generated by the airflow-induced vibration generators 110a, 110b may be transferred, in an amplified way, from the first lid 210 and the second lid 220 closing off the corresponding airflow-induced vibration generators 110a, 110b to the printed circuit board 130 (and thus to the accelerometers 135 electrically and mechanically connected to such printed circuit board 130).

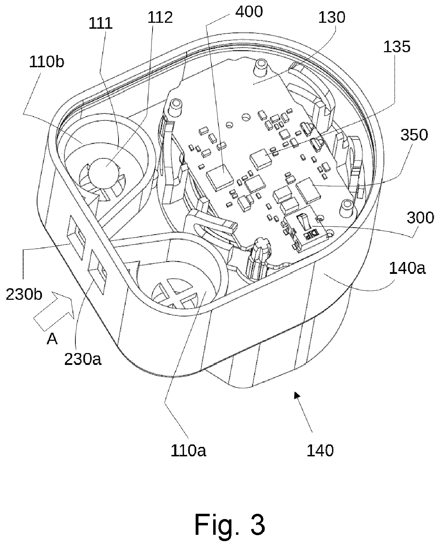

As can be seen in figure 3, the accessory body 140 (and the corresponding airflow-induced vibration generator) may comprise one or more air inlets 230a, 230b configured to receive an air flow, in the direction of the arrow (arrow A), generated by a user by applying suction on the mouthpiece of the inhaler and to draw such air flow to the accessory 100, specifically to the airflow-induced vibration generators 110a, 110b. Particularly, each air inlet 230a, 230b is configured to draw such air flow to corresponding airflow-induced vibration generator 110a, 110b. The air inlets 103a, 103b may have different dimensions. The bigger the dimensions or the number of air inlets, the lower the resistance offered by the inhaler 100 to inhalation. This provides a suitable resistance, by the inhaler, to inhalation. In this particular example, two air inlets 230a, 230b are provided.

The accessory body 140 may further comprise an air outlet (not visible) configured to deliver the air flow, previously received into the accessory 100, via the air inlets 230a, 230b to the user for inhalation.

As commented above, the accessory 100 comprises one or more airflow-induced vibration generators 110a, 110b. In this particular example, two airflow-induced vibration generators 110a, 110b are depicted. The airflow-induced vibration generator 110a may be activated under the influence of the air flow passing through the corresponding air inlet and may be configured to generate mechanical oscillations. The airflow-induced vibration generators may be situated in any part of the chassis of the accessory 100 as long as they are situated near the accelerometer.

Particularly, the airflow-induced vibration generator 110b, may comprise an airflow-induced vibration generator chamber 111 accommodating one or more spinning elements, in this example, one spinning element 112. The spinning element 112 is a component that is capable of motion in response to the airflow passing through the chamber 111 during inhalation by the patient. In this respect, the spinning element 112 may be moved freely within the vibration generator chamber 111. The spinning element 112 is configured to interact with the interior surfaces of the enclosed chamber 111, resulting in the generation of mechanical oscillations.

In some other examples not shown, a guiding pin may be provided to guide the spinning elements within the vibration generator chamber 111. The vibration generator chamber 111 may also comprise elements (not shown) configured to interfere with the motion of the spinning elements such that the vibrations provoked by the spinning elements are increased.

In use, inhalation from a patient via the mouthpiece may cause air flow to pass, in the direction of the arrow (arrow A), through the air inlets 230b to enter into airflow-induced vibration generator chamber 111. Tangential airflow generates a spinning movement of the spinning element 112 contained in the chamber 111. This will generate mechanical oscillations and thus vibration signals. These mechanical oscillations may be transmitted to the printed circuit board 130 (and thus to the accelerometer 135 electrically connected to the printed circuit board) via e.g., the sidewalls of the chassis (not visible in this figure but shown in figure 2: see element 160) and the pins of the internal cover (not visible in this figure but shown in figure 2: see element 200).

The spinning element 112, in this example, is a spherical ball although some other spinning elements may be possible e.g., a bone-shaped spinning element, a T-shaped spinning elements or a helicoid.

In this example, a single air-inlet 230b is connected to corresponding airflow-induced vibration generator chamber 111. However, in some other examples, two or more air inlets may be included connected to corresponding airflow-induced vibration generator chamber 111.

In some other examples not shown, the airflow-induced vibration generator 110b may comprise a toroidal race accommodating a spinning element e.g., a spherical ball which can be moved freely in a circular path under the influence of airflow passing via the air inlet 230b of the airflow-induced vibration generator 11 0b. The air inlet 230b may comprise a smooth, quadrangular hole and may be oriented tangentially to the toroidal race. The interaction of the spherical ball with the toroidal race may generate mechanical oscillations.

To enhance the mechanical oscillations during the circular path of the spherical ball, the vibration generator may further include components placed inside the toroidal race that the spherical ball contacts while moving due to the airflow. Alternatively, the vibration generator may include multiple spherical balls within the toroidal race that interact with both the toroidal race and each other.

In some other examples not shown, the spinning element of the airflow-induced vibration generator may be a propeller (not shown). The propeller may be designed to rotate when airflow is drawn, via the air inlet, into the vibration generator chamber of the airflow-induced vibration generator. In this example, the air inlet may be positioned and oriented perpendicular to the axis of rotation of the propeller.

The airflow-induced vibration generator 110a may comprise the same structure as the airflow-induced vibration generator 110b. However, the airflow-induced vibration generator 110a differs with respect to the airflow-induced vibration generator 110b in the fact that it does not include a spinning element. The airflow-induced vibration generator 110b may thus be a "fake" airflow-induced vibration generator which has been included to decrease the air flow resistance, offered by the inhaler 10, during the inhalation of a user via the mouthpiece and thus does not include spinning elements. In this respect, the lower the airflow resistance of the accessory 100, the higher will be the inspiratory airflow required to displace (and thus make spin) the spinning element.

It is noted that the vibration signal, generated by the airflow-induced vibration generators, may have a Signal-To-Noise ratio (SNR) with a ratio equal or higher than 4:1. This ratio, determined by the Root Mean Square Error (RMSE) of the vibration signal, may become more favorable as the force of the inhalation airflow increases. A higher SNR indicates a stronger and more distinguishable vibrational signal relative to any background noise or interference present in the measurement.

In examples, the (detrended) vibration signal may display a dominant frequency above 100 Hz, specifically ranging between 200 Hz and 400 Hz. The inventors have found that providing a vibration signal focusing on this range, the accessory 100 optimizes its sensitivity to capture the primary vibrations associated with the airflow.

In examples, the spectral power associated with this dominant frequency between 200 Hz and 400 Hz of the vibration signal may be positive and, specifically, at least five times higher than the spectral power of the vibration signal in the absence of external stimuli (noise). This criterion ensures that a significant portion of the signal's energy is concentrated within the desired frequency range, facilitating the accurate interpretation of airflow properties.

In some other examples, a significant portion of the total spectral energy of the vibration signal is concentrated within a 100 Hz bandwidth centered around the dominant frequency. Particularly, at least 50% of the spectral energy of the vibration signal is concentrated within a 100 Hz bandwidth centered around the dominant frequency. More particularly, the 75% of the vibration signal of the spectral energy falls within this bandwidth. This concentration of energy ensures that the most relevant and informative components of the vibrational signal are captured.

The accessory 100 (particularly the monitoring system) may further comprise a detection unit 300 arranged to detect an actuation of the inhaler to dispense a quantity of a formulation to be discharged. For example, the detection unit 300 may comprise a mechanical switch configured to be activated upon actuation of the inhaler, as will be explained later on. The detection unit is configured to generate a first signal upon activation of the detection unit (and thus the actuation of the inhaler). The electronic processing unit 350 may be configured to receive, from the detection unit, the signal indicating that the inhaler has been actuated and thus confirm that the inhaler has been actuated.

The accessory 100 may further comprise an electronic processing unit 350 electrically connected to the printed circuit board 130. The electronic processing unit 350 is configured to receive acceleration signals from the accelerometers 135 and a signal upon activation of the detection unit 300. The reception of a signal from the detection unit may trigger the processing of signals from the accelerometer by the electronic processing unit in order to generate inhalation data. In examples the accelerometer 135 and the electronic processing unit 350 may be kept in a sleeping or low power mode and are activated by a signal from the detection unit 300. This way, the detection unit 300 may allow an optimization in the processing of signals and the battery consumption.

The detection unit 300 may further be configured to generate a signal when an air flow generated by a user, by applying suction on a mouthpiece of the inhaler, is detected. For example, the detection unit 300 may comprise a sensor configured to be activated once a user applies suction on the mouthpiece and airflow is generated. The electronic processing unit 350 is configured to receive, from the detection unit, a signal indicating that an air flow generated by a user by applying suction on a mouthpiece of the inhaler is detected and to confirm that the start of inhalation has been detected.

The electronic processing unit 350 may further be configured to confirm whether the first signal indicating that an air flow generated by a user by applying suction on a mouthpiece of the inhaler is detected has been received before a signal indicating that the inhaler has been actuated. This indicates whether the user is effectively synchronizing his inhalation effort with the inhaler's actuation. Optimal delivery outcomes are generally achieved when the user begins inhaling slightly before actuating the inhaler and continues to inhale for a period after the actuation.

The electronic processing unit 350 may further be configured to identify the time at which the signal indicating the detection of an air flow generated by a user by applying suction on a mouthpiece of the inhaler is received. The electronic processing unit 350 is further configured to identify the time at which the signal indicating that the inhaler has been actuated is received. The electronic processing unit 350 may further be configured to determine a time difference between the reception of the signal indicating that the inhaler has been actuated and the reception of the signal indicating that an air flow has been generated. For example, if the time difference between the signal indicating that an air flow has been generated and then the actuation of the inhaler is within a range of 0.1 to 1 second (specifically not exceeding 0.5 seconds), it may be determined that the user is effectively synchronizing their inhalation effort with the inhaler's actuation.

The processor may also be configured to determine whether a time difference between the time at which the signal indicating that an air flow generated by a user is detected has been received and the time at which this signal is no longer received is within a threshold (or above a threshold). For example, if the time difference following the inhaler's actuation is between 2 seconds and 5 seconds (or above 2.5 seconds), it may be determined that the user has inhaled for an adequate duration.

The electronic processing unit 350 is configured to process the signals from the accelerometer in order to provide inhalation data and to estimate one or more airflow properties based on the vibrations generated by the airflow vibration generators. Such parameters may be e.g., inhaled air volume (the air inhaled during a single inhalation) peak inspiratory flow (the maximum rate of airflow during inhalation), inhalation duration (the length of time of the inhalation), inhalation acceleration (the rate at which the flow of air changes during inhalation).

In examples, the electronic processing unit 350 may further be configured to estimate or evaluate the above-mentioned parameters on the basis of mechanical and/or design properties of the inhaler. Specifically, the electronic processing unit 350 may take into account, to estimate such parameters, properties of the inhaler body 140, the mouthpiece 25, and / or the printed circuit board 130, such as the geometry and/or arrangement of each of these elements. For example, the specific configuration of the inhaler body 140 and/or the air inlets 230a, 230b might be taken into consideration. These properties may be e.g., pre-stored in a memory electrically coupled with the electronic processing unit.

The accessory 100 may further comprise a transmitter-receiver unit 400 electrically coupled to the printed circuit board 130. The transmitter-receiver unit is configured to implement at least one radio communication with one or more external electronic apparatus, for example, a smartphone or a remote server. Such radio communication may use a standard from the short range related protocols communicating over a WPAN (including BLE, Bluetooth 5 and, IEEE 802.15.4, and all their variants) and a WLAN (including IEEE 802.11 and all their variants) or a direct internet connection using long range related protocols (including LoRaWAN, SigFox, EC-GSM, LTE, NB-loT, LTE-M, and all their variants) without the need of an internet gateway. The transmitter-receiver unit may be configured to wirelessly exchange inhalation data generated by the processing unit.

Figures 4a - 4d schematically illustrate a sequence of situations that may occur during the performance of a method to operate a metered-dose inhaler with and installed accessory as hereinbefore described. Same reference numbers denote the same elements. The method is described below with reference to the sequences of situations illustrated by figures 4a-4d.

The figure 4a illustrates an initial situation. In this initial situation, no pressure has been exerted on the top of the inhaler (specifically to the upper surface of the lid 150 of the accessory) in the direction of the arrow (arrow B). As a result, the lid 150 of the accessory is maintained at a first operational position via the elastic elements of the central support. Typically, the lower surface of the lid may rest on an end the elastic elements 155 and thus the lid does not make contact with a lever 300a forming part of the detection unit 300.

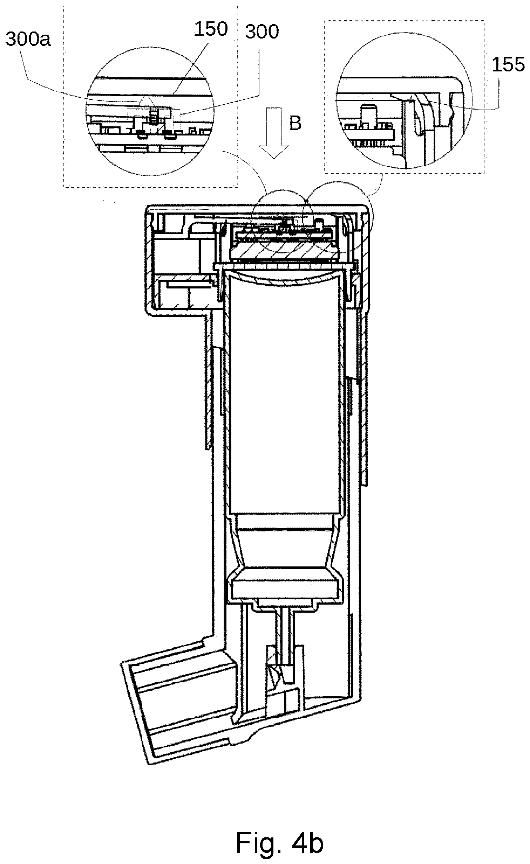

In figure 4b, the top part of the accessory (specifically to the upper surface of the lid 150 of the accessory) has been pressed in the direction of the arrow (arrow B) with a thumb of the user supporting the lower portion of the body of the inhaler. As a result, the elastic elements 155 have been bended and a first sub-group of elements, which includes the lid 15, the accessory body, the chassis (not visible in this figure) and the internal cover (not visible in this figure), has been displaced in the direction of the arrow (arrow B) with respect to a second sub-group of elements of the inhaler, which includes the central support, the printed circuit board and the battery, until an intermediate operational position is reached.

Once the intermediate operational position is reached, the lower part of the lid 150 makes contact with the lever 300a forming part of the detection unit 300 (electrically connected to the PCB) such that a signal is generated by the detection unit 300. This signal may be received by electronic processing unit forming part of the printed circuit board and may trigger the processing of signals from the accelerometer, by the electronic processing unit, in order to generate inhalation data. Basically, the function of the lever 300a and the detection unit 300 is to "wake up" the accessory and inform the accessory that an inhalation might occur or might have occurred within a certain lapse of time from switch activation.

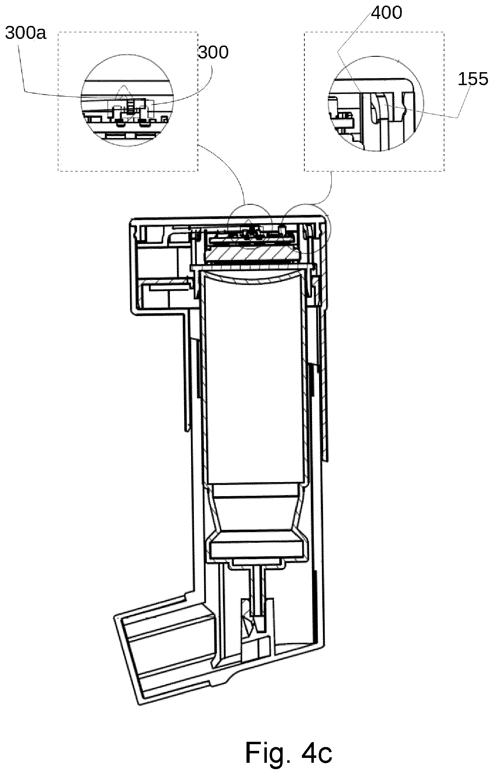

In figure 4c, the top part of the accessory has further been pressed in the direction of the arrow (arrow B), with the thumb of the user, supporting the lower portion of the body. The first sub-group of elements is further displaced with respect to the second sub-group of elements until a lower part of the lid 150 reaches an upper part of the pillars 155 (in this example, four pillars although only one of the pilar is visible in figure 4c) forming part of the central support 180.

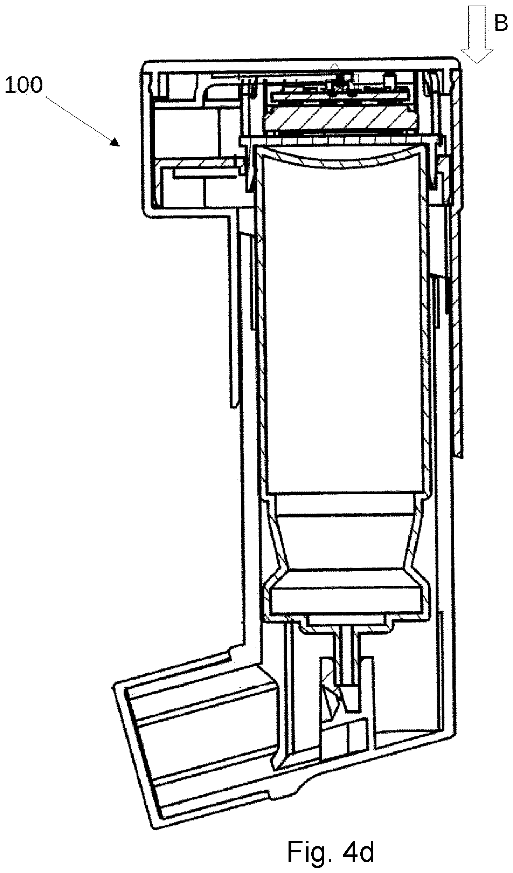

In figure 4d, the top part of the accessory has further been pressed in the direction of the arrow (arrow B), with the thumb of the user, supporting the lower portion of the body. This causes the accessory 100 to be displaced, as a single solid, in the direction of the arrow (arrow B). As a result, a pressure is applied to the upper part of the canister and thus the inhaler is actuated.

Actuation of the inhaler releases a single metered dose of the formulation which contains the medication either dissolved or suspended in the propellant. Breakup of the volatile propellant into droplets, followed by rapid evaporation of these droplets, results in the generation of an aerosol comprising micrometer-sized medication particles that are then inhaled.

In examples, inhalation by a user (which may trigger the spinning of the sphere and therefore the vibrations detected by the accelerometer as hereinbefore described) may occur almost in parallel with the activation of the accessory and the actuation of the inhaler or slightly before.

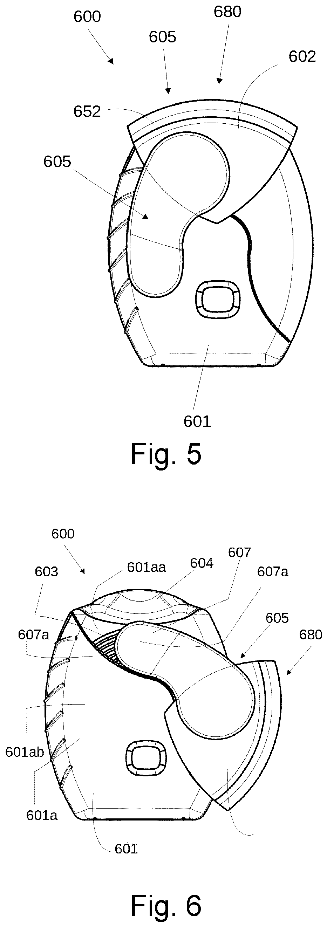

Figure 5 schematically illustrates a perspective view of another example of an inhaler in a closed configuration and another example of an accessory installed on the inhaler. Figure 6 schematically illustrates a perspective view of the inhaler in an open configuration and the same accessory installed on the inhaler.

In this particular example, the inhaler 600 is a dry powder inhaler. This inhaler may be substantially similar to the "ELLIPTA" inhaler. As can be seen in figure 6, the inhaler 600 comprises a body closed by a casing 601 and a mouthpiece 604. The casing 601 comprises an air inlet 603 configured to receive an air flow generated by a user by applying suction on the mouthpiece 604 of the inhaler.

The inhaler 600 further comprises an accessory 605 which may be removably coupled to the inhaler 600 or integrally formed with the inhaler. The accessory 605 is configured for measuring airflow properties of e.g., the air flow directed to the mouth of the user provoked by the inhalation of a user via the mouthpiece 604. The properties are the same as the one described in previous examples.

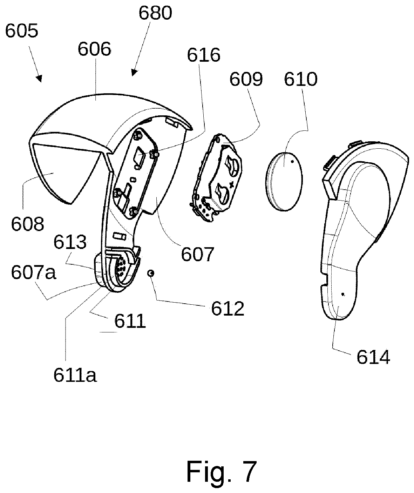

Referring to figure 7, the accessory 605 comprises a sliding cover assembly 680 including an upper curved wall 606, a first sidewall 607 and a second sidewall 608.

The first sidewall 607 may comprise a central opening 616 in which a printed circuit board 609 as hereinbefore described is situated. A battery 610 as hereinbefore described is also provided. The battery 610 may be electrically connected to the printed circuit board 609. The accessory 605 may further comprise one or more airflow-induced vibration generators 611 with an airflow-induced vibration generator chamber 611a accommodating one or more spinning elements 612, as hereinbefore described.

In this example, a single airflow-induced vibration generator 611 is situated at or near a first end 607a of the first sidewall 607. The airflow-induced vibration generator 611 comprises an air inlet 613 configured, in use, to receive an air flow generated by a user by applying suction on the mouthpiece of the inhaler and to draw such air flow to the spinning elements 612. A separate lid or cap 614 for closing off a top surface of the first sidewall 607 of the sliding cover assembly 680 (and thus the accessory) may also be provided.

The sliding cover assembly 680 (and thus the accessory 605) may be pivoted on the casing 601 of the inhaler from a closed position in which the upper curved wall 652 covers the mouthpiece 604, as shown in figure 5, to an open position exposing the mouthpiece 604, as shown in figure 6. The opening movement of sliding cover assembly 680 (and thus the accessory 605) from the closed position shown in Fig. 5 to the open position shown in Fig. 6 may cause the loading of the inhaler 600, i.e. the predisposition of drug blisters for subsequent administration through the inhalation. In examples, the blister with corresponding drug dose may be placed within the casing 601 of the inhaler 600 during manufacturing of such inhaler 600.

Additionally, when the sliding cover assembly 680 (and thus the accessory 605) is in the open position (Fig. 6), the air inlet 603 forming part of the casing 601 is aligned with the air inlet 613 (not visible in this figure but shown in figure 7) of the airflow-induced vibration generator (not visible in this figure but shown in figure 7). As a result, the air inlet 613 of the airflow-induced vibration generator is configured to receive an air flow generated by a user by applying suction on the mouthpiece 604 of the inhaler, via the air inlet 603, and draw such air flow to the spinning element 612. Similarly as in previous examples, the airflow-induced vibration generator may be activated under the influence of the air flow passing through the corresponding air inlet 613 and may be configured to generate mechanical oscillations. The accessory may comprise a monitoring system with a structure and operation as hereinbefore described. In short, the electronic processing unit (see fig. 7) forming part of the printed circuit board 609 may process the acceleration signals emitted by accelerometer sensors (which have been generated based on mechanical oscillations received by the accelerometer sensors) and obtain inhalation data (e.g., estimate one or more airflow properties) based on such received signals.

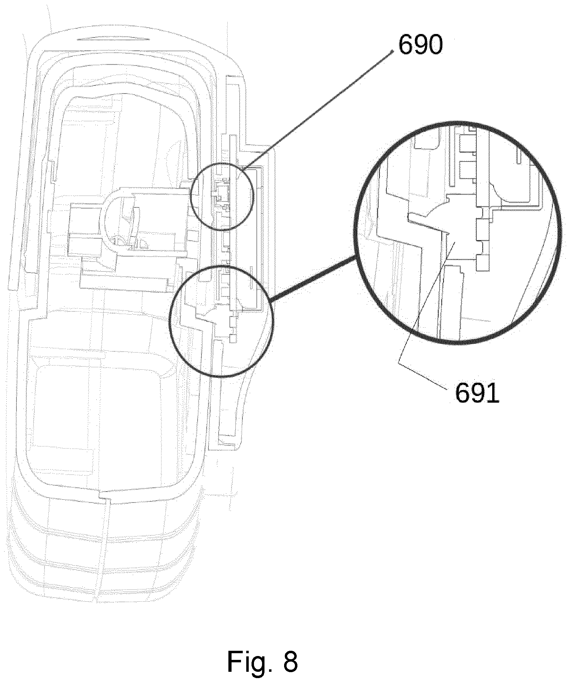

As can be seen in figure 6, the upper surface 601a of the inhaler's casing, onto which the air inlet (not visible in this figure but visible in figure 8) of the accessory 605 slides as the sliding cover assembly (and thus the accessory) is displaced from a closed position to an open position, may comprise different upper surface levels i.e., levels closer or further away with respect to a central part of the inhaler. For example, the surface 601 aa is at different level as compared to surface 601ab.

In order to provide a proper adjustment of the accessory's inlet (not visible in figure 6 but visible in figure 7 with reference number 613) to the different levels of the upper surface of the inhaler's casing, as the sliding cover assembly 680 is displaced from an closed position to an open position, the first end 607a of the first sidewall 607 of the accessory (and thus the accessory's inlet) may be spring loaded against the surface of casing 601 i.e., the first end is held in position by an elastic force. As a result, the first end 607a of the first sidewall 607 of the accessory (and thus the air inlet 613 of the airflow-induced vibration generator) stays suitably pressed against the surface of the case during the sliding of the accessory between the open position and the closed position.

Specifically, the air inlet 613 (not visible in figure 6 but visible in figure 7 with reference number 613) of the airflow-induced vibration generator (not visible in figure 6 but visible in figure 7 with reference number 611) remains suitably pressed against the air inlet 603 of the inhaler, when the accessory is in the open position. This is important in order to assure that there is no air leakage in the transfer of air between corresponding air inlets.

In examples, the above-commented proper adjustment can be achieved using one or more spring elements e.g. steel springs or plastic springs.

In some other examples, the first end 607a of the first sidewall 607 of the accessory may be designed using appropriate flexible materials such that first end 607a of the first sidewall 607 of the accessory (and thus the air inlet 613 of the airflow-induced vibration generator 611) stays suitably pressed against the surface of the case during the sliding of the accessory between the open position and the closed position. Appropriate materials may have good elastic properties and high yield strength. The material may be a metal e.g. steel or plastic resins e.g., polyoxymethylene.

As can be seen in figure 8, the accessory 100 may further comprise an attachment detection unit 690 arranged to detect the fact that the accessory has been attached to the inhaler. For example, the detection unit 690 may comprise a mechanical switch configured to be activated upon attachment of the accessory to the inhaler. In figure 8, the attachment detection unit 690 is shown activated. The attachment detection unit 690 is configured to generate a signal once the accessory has been attached to the inhaler. The signal may be processed by the processing unit.

The accessory may further comprise an open / closed detection unit 691 arranged to detect that the accessory has been situated in an opened or in a closed position. For example, the detection unit may comprise a mechanical switch configured to be activated (i.e. the switch is pressed against the upper surface of the casing of the inhaler: see figure 8) when the accessory is in a closed position and the switch is configured to be deactivated (i.e. the switch is not pressed against the upper surface of the casing of the inhaler) when the accessory is in a opened position. The open / closed detection unit 691 is configured to generate a signal once the accessory is pressed against the upper surface of the casing of the inhaler.

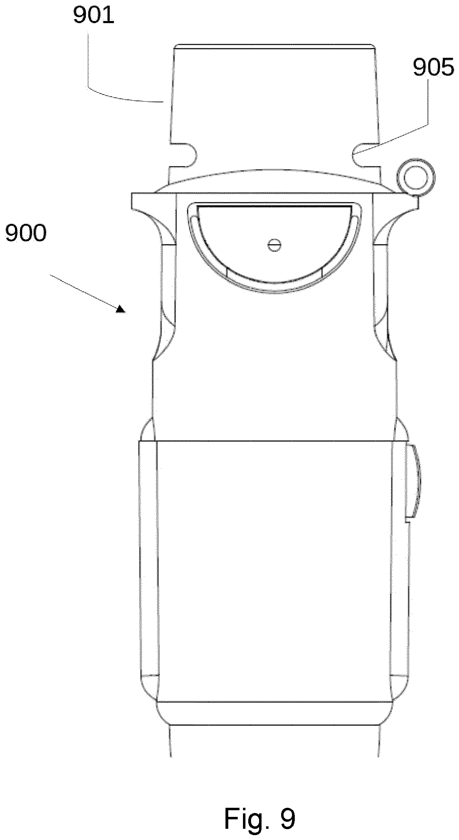

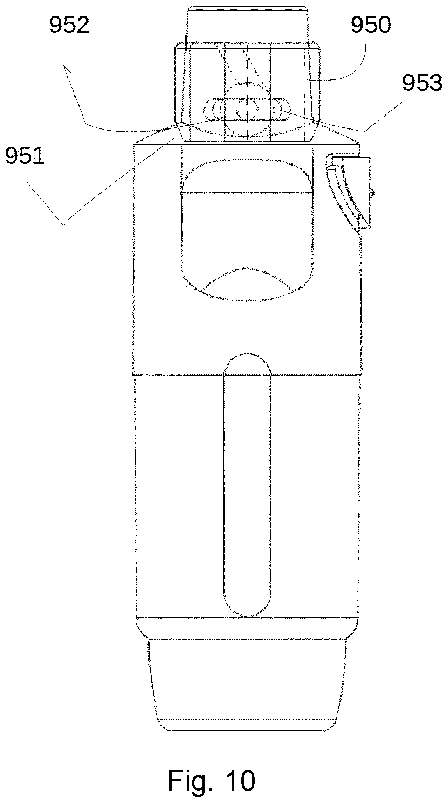

Figure 9 schematically illustrates a perspective view of yet another inhaler. Figure 10 schematically illustrates a perspective view of the inhaler and an accessory installed on the inhaler.

In this particular example, the inhaler 900 is a soft mist inhaler. This inhaler corresponds to a nebulizer for nebulizing a fluid (not visible), particularly a highly effective pharmaceutical composition or the like. The nebulizer is constructed, in particular, as a portable inhaler.

When the fluid (not visible), preferably a liquid, more particularly a pharmaceutical composition, is nebulized, an aerosol is formed which can be breathed in or inhaled by a user (not shown). Usually, the inhaling is done at least once a day, more particularly, several times a day, preferably at set intervals. The nebulizer has an insertable and preferably exchangeable container (not visible) containing the fluid, which forms a reservoir for the fluid which is to be nebulized.

The nebulizer has a pressure generator for conveying and nebulizing the fluid, particularly in a preset and optionally adjustable dosage amount. A user (not shown) can inhale the aerosol, while an air supply can be sucked into the mouthpiece 901 through at least one air supply opening 905.

An accessory as hereinbefore described may be provided. A cap (not shown) for covering the mouthpiece 901 may be removed before installing the accessory. Once the cap has been removed, the accessory 950 may be installed to the inhaler at or near the mouthpiece and situated on a support 951.

Similarly as in previous examples, the accessory 950 comprises an airflow-induced vibration generator 952 including an air inlet 953 configured, in use, to receive an air flow generated by a user, via the air supply opening 905, by applying suction on the mouthpiece of the inhaler and to draw such air flow to the corresponding spinning elements. The corresponding air inlets may be aligned. These mechanical vibrations generated by the spinning elements may be transmitted to the printed circuit board (not shown) and to the accelerometer sensors. Electronic processor may receive from the accelerometer sensor, the generated acceleration signals and extract inhalation data from the received acceleration signal.

For reasons of completeness, various aspects of the present disclosure are set out in the following numbered clauses:
Clause 1. An accessory for obtaining inhalation data from an inhaler, wherein the accessory is integrally formed with or attached to the inhaler, the accessory comprising:
   - one or more airflow-induced vibration generators configured to generate mechanical oscillations,
   - a monitoring system comprising:
      ∘ one or more accelerometer sensors configured to:
         ▪ receive, from the airflow-induced vibration generators, the generated mechanical oscillations,
         ▪ generate acceleration signals based on the received mechanical oscillations,
      ∘ an electronic processing unit electrically connected to the accelerometer sensors, configured to:
         ▪ receive, from the accelerometer sensors, the generated acceleration signals,
         ▪ extract inhalation data from the received acceleration signal.
Clause 2. An accessory according to clause 1, wherein inhalation data is one or more of the following:
   - inhalation flow rate;
   - inhalation volume;
   - peak inhalation flow rate;
   - inhalation duration.
Clause 3. An accessory according to any of clauses 1 - 2, wherein the accessory comprises an accessory body having one or more sidewalls extending between a top and a bottom of the accessory body, and a space being formed in an interior of the container body between the sidewalls.
Clause 4. An accessory according to clause 3, wherein the accessory comprises a chassis, in use, disposed in the space formed in the interior of the accessory body, wherein the chassis comprises an annular top platform including a central opening configured to receive a central support, and one or more outer sidewalls, wherein the airflow-induced vibration generators form part of the chassis.
Clause 5. An accessory according to any of clauses 1 - 4, wherein at least one of the airflow-induced vibration generators comprise an airflow-induced vibration generator chamber including one or more spinning elements, wherein the spinning elements are configured to interact with interior surfaces of the airflow-induced vibration generator chamber such that mechanical oscillations are generated.
Clause 6. An accessory according to clause 5, wherein the airflow-induced vibration generators comprise one or more air inlets configured to receive an air flow generated by a user by applying suction on a mouthpiece of the inhaler, wherein the inlets are further configured to draw the received air flow to the interior of the corresponding airflow-induced vibration generator chamber such that the spinning elements are set in motion to interact with the interior surfaces of airflow-induced vibration generator chamber.
Clause 7. An accessory according to any of clauses 1-6, wherein at least one of the airflow-induced vibration generators does not include spinning elements.
Clause 8. An accessory according to any of clauses 3-7, wherein the monitoring system further comprises a printed circuit board mechanically coupled to the accessory body, wherein the electronic processing unit and the accelerometer sensors are electrically connected to the printed circuit board, wherein the printed circuit board is configured to transmit mechanical oscillations from the inhaler body to the accelerometer sensors.
Clause 9. An accessory according to clause 8, wherein the coupling between the printed circuit board and the accessory body is rigid and / or elastic.
Clause 10. An accessory according to any of clauses 5-9, further comprising an internal cover configured to at least partially closing off the vibration generator chamber of the airflow-induced vibration generators.
Clause 11. An accessory according to clause 10, wherein the internal cover extends from a first end to a second end, wherein the second end comprises one or more pins configured to be mechanically attached to the printed circuit board such that mechanical oscillations are transferred from the airflow-induced vibration generators to the printed circuit board.
Clause 12. An accessory according to any of clauses 1 - 11, wherein the monitoring system comprises a detection unit configured to:
   generate a signal when the inhaler is actuated to dispense a quantity of a formulation to be discharged, and the processing unit is configured to:
   receive, from the detection unit, the signal indicating that the inhaler has been actuated.
Clause 13. An accessory according to any of clauses 1 - 12, wherein the monitoring system comprises a detection unit configured to:
   generate a signal when an air flow generated by a user by applying suction on a mouthpiece of the inhaler is detected, and the processing unit is configured to:
   receive, from the detection unit, the signal indicating that an air flow generated by a user by applying suction on a mouthpiece of the inhaler is detected,
Clause 14. An accessory according to clause 13 when dependent on clause 12, wherein the processing unit is configured to:
   identify the time at which the signal indicating the detection of an air flow generated by a user by applying suction on a mouthpiece of the inhaler is received,
   identify the time at which the signal indicating that the inhaler has been actuated is received,
   determine a time difference between the time at which the signal indicating actuation of the inhaler has been received and the time at which the signal indicating detection of an air flow has been received.
Clause 15. An accessory according to clause 14, wherein the processing unit is further configured to:
   determine whether the time difference is within a time interval, specifically the time interval falls within a range of 0.1 to 1 second.
Clause 16. An accessory according to any of clauses 1 - 2 and 5-6, wherein the accessory comprises a sliding cover assembly including an upper curved wall, a first sidewall and a second sidewall.
Clause 17. An accessory according to clause 16, wherein the monitoring system further comprises a printed circuit board mechanically coupled to the first sidewall, wherein the electronic processing unit and the accelerometer sensors are electrically connected to the printed circuit board, wherein the printed circuit board is configured to transmit vibration signals from the first sidewall to the accelerometer sensors.
Clause 18. An accessory according to any of clauses 16 - 17, wherein the airflow-induced vibration generators are situated at or near a first end of the first sidewall.
Clause 19. An accessory according to any of clauses 16 - 18, wherein the sliding cover assembly is configured to be displaced between a closed position in which the upper curved wall covers a mouthpiece of the inhaler and an open position in which the upper curved wall does not cover a mouthpiece of the inhaler.
Clause 20. An accessory according to clause 19 when dependent on clause 6, wherein, when the sliding cover is in an open position, the air inlet of the corresponding airflow-induced vibration generator is aligned with an air inlet situated in a casing of the inhaler.
Clause 21. An accessory according to clause 20, wherein the corresponding air inlet of the airflow-induced vibration generators is configured to receive an air flow generated by a user by applying suction on the mouthpiece of the inhaler, via the air inlet situated in the casing of the inhaler, and to draw such air flow to the airflow-induced vibration generators.
Clause 22. An accessory according to any of clauses 18 - 21, wherein the first end of the first sidewall of the accessory is spring loaded against a surface of an inhaler's casing.
Clause 23. An accessory according to any of clauses 16 - 22, wherein the monitoring system further comprises:
   an attachment detection unit configured to generate a signal once the accessory has been attached to the inhaler and
   the electronic processing unit is configured to receive the signal generated by the attachment detection unit such that the attachment of the of the accessory is detected.
Clause 24. An accessory according to any of clauses 16 - 23, wherein the monitoring system further comprises:
   an opening detection unit configured to generate a signal once the sliding cover is in a closed position and
   the electronic processing unit is configured to receive the signal generated by the attachment detection unit such that the closed position of the sliding cover is detected.
Clause 25. A kit comprising:
   an accessory according to any of clauses 1 - 24,
   an inhaler.
Clause 26. A kit according to clause 25, wherein the inhaler is one of the following: a metered-dose inhaler, a dry powder inhaler, or a soft mist inhaler.

Although only a number of examples have been disclosed herein, other alternatives, modifications, uses, and/or equivalents thereof are possible. Furthermore, all possible combinations of the described examples are also covered. Thus, the scope of the present disclosure should not be limited by particular examples, but should be determined only by a fair reading of the claims that follow.

## Claims

1. An accessory for obtaining inhalation data from an inhaler, wherein the accessory is integrally formed with or attached to the inhaler, the accessory comprising:
- one or more airflow-induced vibration generators configured to generate mechanical oscillations,
- a monitoring system comprising:
∘ one or more accelerometer sensors configured to:
▪ receive, from the airflow-induced vibration generators, the generated mechanical oscillations,
▪ generate acceleration signals based on the received mechanical oscillations,
∘ an electronic processing unit electrically connected to the accelerometer sensors, configured to:
▪ receive, from the accelerometer sensors, the generated acceleration signals,
▪ extract inhalation data from the received acceleration signal.

2. An accessory according to claim 1, wherein the accessory comprises an accessory body having one or more sidewalls extending between a top and a bottom of the accessory body, and a space being formed in an interior of the container body between the sidewalls.

3. An accessory according to claim 2, wherein the accessory comprises a chassis, in use, disposed in the space formed in the interior of the accessory body, wherein the chassis comprises an annular top platform including a central opening configured to receive a central support, and one or more outer sidewalls, wherein the airflow-induced vibration generators form part of the chassis.

4. An accessory according to any of claims 1-3, wherein at least one of the airflow-induced vibration generators comprise an airflow-induced vibration generator chamber including one or more spinning elements, wherein the spinning elements are configured to interact with interior surfaces of the airflow-induced vibration generator chamber such that mechanical oscillations are generated.

5. An accessory according to claim 4, wherein the airflow-induced vibration generators comprise one or more air inlets configured to receive an air flow generated by a user by applying suction on a mouthpiece of the inhaler, wherein the inlets are further configured to draw the received air flow to the interior of the corresponding airflow-induced vibration generator chamber such that the spinning elements are set in motion to interact with the interior surfaces of airflow-induced vibration generator chamber.

6. An accessory according to any of claims 1-5, wherein at least one of the airflow-induced vibration generators does not include spinning elements.

7. An accessory according to any of claims 2-6, wherein the monitoring system further comprises a printed circuit board mechanically coupled to the accessory body, wherein the electronic processing unit and the accelerometer sensors are electrically connected to the printed circuit board, wherein the printed circuit board is configured to transmit mechanical oscillations from the inhaler body to the accelerometer sensors.

8. An accessory according to any of claims 4-7, further comprising an internal cover configured to at least partially closing off the vibration generator chamber of the airflow-induced vibration generators, wherein the internal cover extends from a first end to a second end, wherein the second end comprises one or more pins configured to be mechanically attached to the printed circuit board such that mechanical oscillations are transferred from the airflow-induced vibration generators to the printed circuit board.

9. An accessory according to any of claims 1 and 4-5, wherein the accessory comprises a sliding cover assembly including an upper curved wall, a first sidewall and a second sidewall.

10. An accessory according to claim 9, wherein the monitoring system further comprises a printed circuit board mechanically coupled to the first sidewall, wherein the electronic processing unit and the accelerometer sensors are electrically connected to the printed circuit board, wherein the printed circuit board is configured to transmit vibration signals from the first sidewall to the accelerometer sensors.

11. An accessory according to any of claims 9 - 10, wherein the airflow-induced vibration generators are situated at or near a first end of the first sidewall.

12. An accessory according to any of claims 9 - 11, wherein the sliding cover assembly is configured to be displaced between a closed position in which the upper curved wall covers a mouthpiece of the inhaler and an open position in which the upper curved wall does not cover a mouthpiece of the inhaler.

13. An accessory according to claim 12 when dependent on claim 5, wherein, when the sliding cover is in an open position, the air inlet of the corresponding airflow-induced vibration generator is aligned with an air inlet situated in a casing of the inhaler, wherein the corresponding air inlet of the airflow-induced vibration generators is configured to receive an air flow generated by a user by applying suction on the mouthpiece of the inhaler, via the air inlet situated in the casing of the inhaler, and to draw such air flow to the airflow-induced vibration generators.

14. An accessory according to any of claims 11 - 13, wherein the first end of the first sidewall of the accessory is spring loaded against a surface of an inhaler's casing.

15. An accessory according to any of claims 9 - 14, wherein the monitoring system further comprises:
an attachment detection unit configured to generate a signal once the accessory has been attached to the inhaler and
the electronic processing unit is configured to receive the signal generated by the attachment detection unit such that the attachment of the of the accessory is detected, wherein the monitoring system further comprises:
an opening detection unit configured to generate a signal once the sliding cover is in a closed position and
the electronic processing unit is configured to receive the signal generated by the attachment detection unit such that the closed position of the sliding cover is detected.
